# EUROPEAN PATENT APPLICATION

(11) **EP 2 821 074 A1**
(43) Date of publication of application: **07.01.2015**
(21) Application number: 13747103.3
(22) Date of filing: 07.02.2013
(51) Int. Cl.: A61K 38/00, A61P 9/10, A61P 43/00

(54) **OXIDIZED LDL INHIBITOR**

(30) Priority: 10.02.2012 JP 2012027793
(71) Applicant: National Cerebral and Cardiovascular Center, Suita-shi Osaka 565-8565 (JP)
(72) Inventor: SAWAMURA, Tatsuya, Suita-shi Osaka 565-8565 (JP); KAKINO, Akemi, Suita-shi Osaka 565-8565 (JP)
(74) Representative: Bittner, Thomas L.
(86) International application number: PCT/JP2013/052939
(87) International publication number: WO 2013/118839

(57) **Abstract**

The present invention provides an oxidized LDL inhibitor that is capable of (i) binding specifically to oxidized LDL, (ii) suppressing the physiological activity of oxidized LDL, and (iii) inhibiting the uptake of oxidized LDL into cells. The oxidized LDL inhibitor according to the present invention contains a Del-1 protein as an active ingredient. The Del-1 protein consists, for example, of the amino acid sequence of SEQ ID NO: 1.

SEQUENCE LISTING

<110>  NATIONAL CEREBRAL AND CARDIOVASCULAR CENTER

<120>  Oxidized low-density lipoprotein inhibitor

<130>  NC1304

<150>  JP 2012-027793
<151>  2012-02-10

<160>  2

<170>  PatentIn version 3.5

<210>  1
<211>  480
<212>  PRT
<213>  Homo sapiens

<400>  1

Met Lys Arg Ser Val Ala Val Trp Leu Leu Val Gly Leu Ser Leu Gly
1               5                   10                  15

Val Pro Gln Phe Gly Lys Gly Asp Ile Cys Asp Pro Asn Pro Cys Glu
            20                  25                  30

Asn Gly Gly Ile Cys Leu Pro Gly Leu Ala Asp Gly Ser Phe Ser Cys
        35                  40                  45

Glu Cys Pro Asp Gly Phe Thr Asp Pro Asn Cys Ser Ser Val Val Glu
    50                  55                  60

Val Ala Ser Asp Glu Glu Glu Pro Thr Ser Ala Gly Pro Cys Thr Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Val Glu Pro Cys
        115                 120                 125

Lys Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala

165                 170                 175

Ser Ser Thr His Arg Ala Leu Phe Gly Leu Gln Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Asp Met Val Phe Arg
            260                 265                 270

Gly Asn Ile Asp Asn Asn Thr Pro Tyr Ala Asn Ser Phe Thr Pro Pro
        275                 280                 285

Ile Lys Ala Gln Tyr Val Arg Leu Tyr Pro Gln Val Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Ile Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val
    370                 375                 380

Pro Thr Lys Val Thr Gly Ile Ile Thr Gln Gly Ala Lys Asp Phe Gly
385                 390                 395                 400

His Val Gln Phe Val Gly Ser Tyr Lys Leu Ala Tyr Ser Asn Asp Gly
                405                 410                 415

Glu His Trp Thr Val Tyr Gln Asp Glu Lys Gln Arg Lys Asp Lys Val
            420                 425                 430

Phe Gln Gly Asn Phe Asp Asn Asp Thr His Arg Lys Asn Val Ile Asp
        435                 440                 445

Pro Pro Ile Tyr Ala Arg His Ile Arg Ile Leu Pro Trp Ser Trp Tyr
    450                 455                 460

Gly Arg Ile Thr Leu Arg Ser Glu Leu Leu Gly Cys Thr Glu Glu Glu
465                 470                 475                 480

<210>  2
<211>  1443
<212>  DNA
<213>  Homo sapiens

<400>  2
atgaagcgct cggtagccgt ctggctcttg gtcgggctca gcctcggtgt cccccagttc      60

ggcaaaggtg atatttgtga tcccaatcca tgtgaaaatg gaggtatctg tttgccagga     120

ttggctgatg gttccttttc ctgtgagtgt ccagatggct tcacagaccc caactgttct     180

agtgttgtgg aggttgcatc agatgaagaa gaaccaactt cagcaggtcc ctgcactcct     240

aatccatgcc ataatggagg aacctgtgaa ataagtgaag cataccgagg ggatacattc     300

ataggctatg tttgtaaatg tccccgagga tttaatggga ttcactgtca gcacaacata     360

aatgaatgcg aagttgagcc ttgcaaaaat ggtggaatat gtacagatct tgttgctaac     420

tattcctgtg agtgcccagg cgaatttatg ggaagaaatt gtcaatacaa atgctcaggc     480

ccactgggaa ttgaaggtgg aattatatca aaccagcaaa tcacagcttc ctctactcac     540

cgagctcttt ttggactcca aaaatggtat ccctactatg cacgtcttaa taagaagggg     600

cttataaatg cgtggacagc tgcagaaaat gacagatggc cgtggattca gataaatttg     660

caaaggaaaa tgagagttac tggtgtgatt acccaaggag ccaagaggat tggaagccca     720

gagtatataa aatcctacaa aattgcctac agtaatgatg gaaagacttg ggcaatgtac     780

aaagtgaaag gcaccaatga agacatggtg tttcgtggaa acattgataa caacactcca     840

tatgctaact ctttcacacc ccccataaaa gctcagtatg taagactcta tccccaagtt     900

tgtcgaagac attgcacttt gcgaatggaa cttcttggct gtgaactgtc gggttgttct     960

gagcctctgg gtatgaaatc aggacatata caagactatc agatcactgc ctccagcatc    1020

ttcagaacgc tcaacatgga catgttcact tgggaaccaa ggaaagctcg gctggacaag    1080

caaggcaaag tgaatgcctg gacctctggc cacaatgacc agtcacaatg gttacaggtg    1140

gatcttcttg ttccaaccaa agtgactggc atcattacac aaggagctaa agattttggt    1200

catgtacagt ttgttggctc ctacaaactg gcttacagca atgatggaga acactggact    1260

gtataccagg atgaaaagca aagaaaagat aaggttttcc agggaaattt tgacaatgac    1320

actcacagaa aaaatgtcat cgaccctccc atctatgcac gacacataag aatccttcct    1380

tggtcctggt acgggaggat cacattgcgg tcagagctgc tgggctgcac agaggaggaa    1440

tga                                                                  1443

## Description

### Technical Field

The present invention relates to an oxidized LDL inhibitor that is capable of (i) binding specifically to oxidized LDL, (ii) inhibiting the uptake of oxidized LDL into cells, and (iii) suppressing an oxidized-LDL-dependent cellular reaction. The oxidized LDL inhibitor according to the present invention is capable of acting on oxidized LDL as a target and suppressing interaction between oxidized LDL and an oxidized LDL receptor.

### Background Art

Oxidized LDL, which is produced by the oxidization of low-density lipoprotein (hereinafter referred to as "LDL"), is known to function to promote arteriosclerosis, and an anti-atherogenic effect can be brought about by reducing the level of oxidized LDL in blood. For example, a prophylactic against arteriosclerosis has been disclosed whose active ingredient is pine bark extract containing less than 95% by weight of proanthocyanidin in dry weight (e.g., see Patent Literature 1). The pine bark extract is highly effective in suppressing the oxidization of lipids in blood vessels, and therefore, the level of oxidized LDL in blood can be reduced by taking the pine bark extract.

Meanwhile, the promoting effect of oxidized LDL on arteriosclerosis is considered to be caused by the uptake of oxidized LDL into vascular endothelial cells through lectin-like oxidized LDL receptor (LOX-1; lectin-like oxidized low-density lipoprotein receptor-1). LOX-1, which was identified as a vascular endothelial oxidized LDL receptor, is currently known as a factor that promotes cardiovascular diseases such as inflammation, arteriosclerosis, thrombus, myocardial infarction, and post-catheterization vascular restenosis. That is, an anti-atherogenic effect can also be brought about by suppressing the binding of oxidized LDL to LOX-1 and the uptake of oxidized LDL into the vascular endothelial cells.

Known as prophylactics having such an inhibitory effect on LOX-1 are as follows: a composition having LOX-1 antagonistic action (e.g., see Patent Literature 2), which contains, as an active ingredient, plant extract of *Pteridium aquilinum,* a class of *Dennstaedtiaceae;* an arteriosclerosis inhibitor having LOX-1 antagonistic action (e.g., see Patent Literature 3), which contains, as an active ingredient, extract of a rutaceous plant; a lectin-like oxidized LDL receptor inhibiting pharmaceutical product (e.g., see Patent Literature 4) whose active ingredient is procyanidin composed of three or more identical monomers having LOX-1 antagonistic action; etc.

Incidentally, developmental endothelial locus-1 (hereinafter referred to as "Del-1") is known as one of proteins expressed and secreted in vascular endothelial cells or in macrophages. Del-1 is known to have the following features:
(1) Del-1 is highly expressed in the brain or in lung tissue (e.g., see Non-patent Literature 1);
(2) Del-1 binds to phosphatidyl serine (PS) in the C1 and C2 domains at the C-terminus of Del-1 to promote phagocytosis of apoptotic cells by macrophages (e.g., see Non-patent Literature 2);
(3) Del-1 promotes angiogenesis (e.g., see Non-patent Literature 3);
(4) Del-1 suppresses adhesion between leukocytes and endothelial cells (anti-inflammatory effect) (e.g., see Non-patent Literature 1); and
(5) Expression of Del-1 is induced in an injured vessel or under a hypoxic condition (e.g., see Non-patent Literatures 4 and 5).

### Citation List

### Patent Literatures

Patent Literature 1
   Japanese Patent Application Publication, Tokukai, No. 2005-23032 (Publication Date: January 27, 2005)
Patent Literature 2
   Japanese Patent Application Publication, Tokukai, No. 2007-297381 (Publication Date: November 15, 2007)
Patent Literature 3
   Japanese Patent Application Publication, Tokukai, No. 2007-320956 (Publication Date: December 13, 2007)
Patent Literature 4
   Japanese Patent Application Publication, Tokukai, No. 2011-6326 (Publication Date: January 13, 2011)

### Non-patent Literatures

Non-patent Literature 1
   Choi EY et al. Del-1, an endogenous leukocyte-endothelial adhesion inhibitor, limits inflammatory cell recruitment. Science 2008, Vol. 322, no. 5904, p. 1101-1104.
Non-patent Literature 2
   Hanayama R et al. Expression of developmental endothelial locus-1 in a subset of macrophages for engulfment of apoptotic cells. The Journal of immunology 2004, vol. 172, no. 6, p. 3876-3882.
Non-patent Literature 3
   Penta K et al. Dell induces integrin signaling and angiogenesis by ligation of alphaVbeta3. J Biol. Chem., 1999, Vol. 274, no. 16, p. 11101-11109.
Non-patent Literature 4
   Rezaee M et al. Dell mediates VSMC adhesion, migration, and proliferation through interaction with integrin alpha(v)beta(3). Am J Physiol Hert Circ Physiol 2002, vol. 282, no. 5, p. 1924-1932.
Non-patent Literature 5
   Scheurer SB et al. Modulation of gene expression by hypoxia in human umbilical cord vein endothelial cells: A transcriptomic and proteomic study. Proteomics 2004, vol. 4, no. 9, p. 1737-1760.
Non-patent Literature 6
   Steinbrecher UP. Receptors for oxidized low density lipoprotein. Biochim Biophys Acta. 1999, vol. 436, no. 3, p. 279-298.

### Summary of Invention

### Technical Problem

The uptake of oxidized LDL into cells is known to be performed through a group of oxidized LDL receptors called scavenger receptors, as well as LOX-1 (see Non-patent Literature 6). Existing examples of oxidized LDL receptors other than LOX-1 include SR-A (scavenger receptor-A I/II: scavenger receptor A), CD36, SR-BI (scavenger receptor class B type 1), etc.

Since the drugs described in Patent Literatures 2 to 4 are LOX-1 antagonists, they are effective against the uptake of oxidized LDL into cells through LOX-1, but may not necessarily be effective against the uptake of oxidized LDL into cells through another type of oxidized LDL receptor. For this reason, the drugs described in Patent Literatures 2 to 4 alone have sometimes been insufficient to serve as effective means for treating arteriosclerotic disease.

In order to solve the foregoing problems, it is necessary to prepare antagonists against various types of oxidized LDL receptors, respectively, or to prepare an effective single antagonist against all types of oxidized LDL receptor. However, such an antagonist has yet to be found.

The present invention was therefore intended to, instead of finding an antagonist against an oxidized LDL receptor as a target, find an oxidized LDL inhibitor that is capable of acting on oxidized LDL per se as a target and inhibiting the binding of oxidized LDL to an oxidized LDL receptor.

### Solution to Problem

The inventors of the present invention diligently studied to solve the foregoing problems, and found that Del-1, which is a protein that is secreted in vascular endothelial cells or in macrophages, is capable of (i) binding specifically to oxidized LDL, (ii) inhibiting the uptake of oxidized LDL into cells, and (iii) suppressing an oxidized-LDL-dependent cellular reaction, and finally accomplished the present invention based on the finding. That is, the present invention encompasses the following inventions:
An oxidized LDL inhibitor according to the present invention contains a Del-1 (developmental endothelial locus-1) protein as an active ingredient.

In the oxidized LDL inhibitor according to the present invention, the Del-1 protein may be (a) a protein consisting of the amino acid sequence of SEQ ID NO: 1 or (b) a protein (i) consisting of an amino acid sequence, in which one or more amino acids are substituted, deleted, inserted, or added in the amino acid sequence of SEQ ID NO: 1, and (ii) having oxidized LDL inhibitory activity.

Furthermore, the present invention also encompasses a pharmaceutical composition containing an oxidized LDL inhibitor according to the present invention.

### Advantageous Effects of Invention

An oxidized LDL inhibitor of the present invention are not targeted at an oxidized LDL receptor like a drug known in the art, but binds to oxidized LDL per se as a target and inhibits the uptake of oxidized LDL into cells. For this reason, the oxidized LDL inhibitor of the present invention is believed to be able to inhibit the uptake of oxidized LDL into cells through any type of oxidized LDL receptor. As such, the oxidized LDL inhibitor of the present invention can serve as effective means for treating or preventing arteriosclerotic diseases that are caused by oxidized LDL.

More surprisingly, the oxidized LDL inhibitor of the present invention makes it possible to suppress an oxidized-LDL-dependent cellular reaction (e.g., activation of NFκB and SRF).

These effects of Del-1 contained as an active ingredient in the oxidized LDL inhibitor of the present invention have never been known before, and have newly been found by the inventors of the present invention. For this reason, even persons skilled in the art cannot easily accomplish the present invention, and the effects that are brought about by the present invention are beyond expectations of persons skilled in the art.

### Brief Description of Drawings

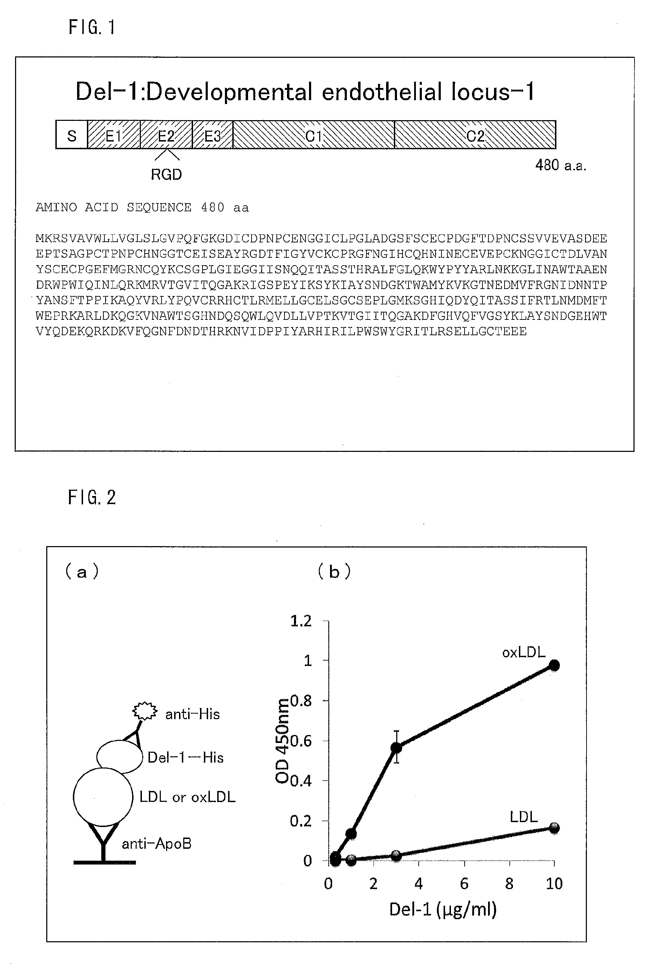

Fig. 1 is a diagram showing a structure and amino acid sequence of Del-1.
Fig. 2 is a set of drawings (a) and (b), (a) being a conceptual diagram explaining a principle of ELISA detection of Del-1 binding to LDL or oxidized LDL, (b) being a graph showing results of the ELISA detection of Del-1 binding to LDL or oxidized LDL.

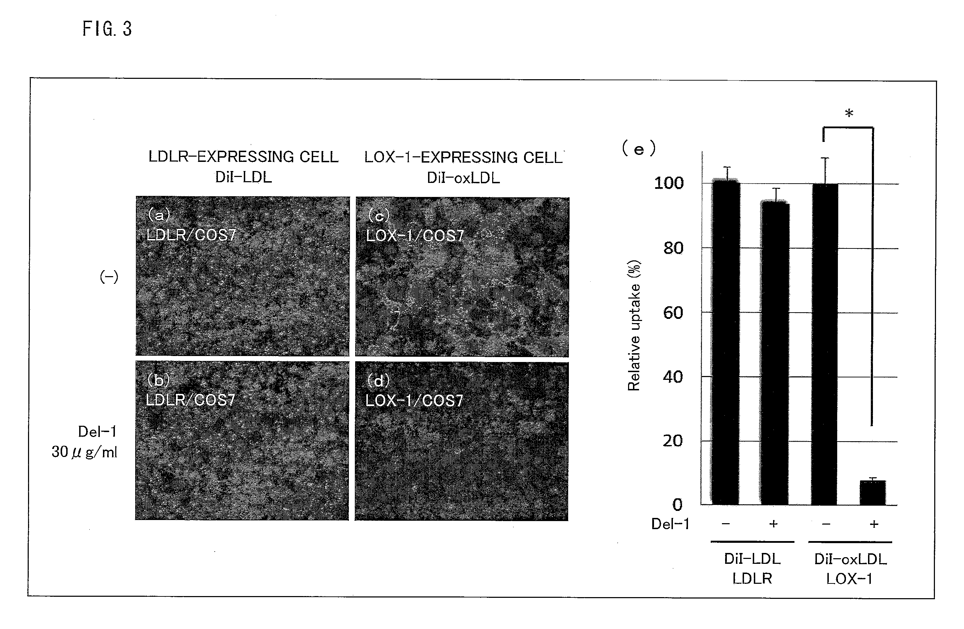

Fig. 3 is a set of drawings (a) through (e) showing results of assessment of the effect of Del-1 on the uptake of oxidized LDL or LDL into LDL-receptor-expressing cells or LOX-1-expressing cells, (a) being a fluorescence micrograph of cells produced by the uptake of DiI-labeled LDL into LDL-receptor-expressing cells in the absence of Del-1, (b) being a fluorescence micrograph of cells produced by the uptake of DiI-labeled LDL into LDL-receptor-expressing cells in the presence of Del-1 (30 µg/ml), (c) being a fluorescence micrograph of cells produced by the uptake DiI-labeled oxidized LDL into LOX-1-expressing cells in the absence of Del-1, (d) being a fluorescence micrograph of cells produced by the uptake of DiI-labeled oxidized LDL into LOX-1-expressing cells in the presence of Del-1 (30 µg/ml), (e) being a graph showing the fluorescence intensity of cells produced by the uptake of DiI-labeled LDL into LDL-receptor-expressing cells in the absence of Del-1 or in the presence of Del-1 (30 µg/ml) and the fluorescence intensity of cells produced by the uptake of DiI-labeled oxidized LDL into LOX-1-expressing cells in the absence of Del-1 or in the presence of Del-1 (30 µg/ml).

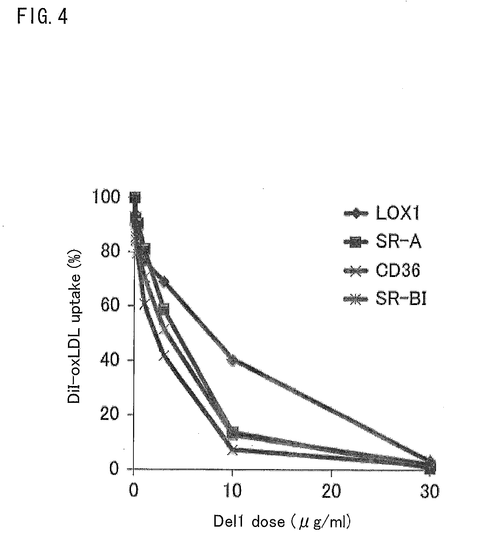

Fig. 4 is a graph showing results of examination of whether or not Del-1 is capable of inhibiting the uptake of oxidized LDL into various types of oxidized-LDL-receptor-expressing cells (LOX-1-expressing cells, SR-A-expressing cells, CD36-expressing cells, SR-BI-expressing cells).

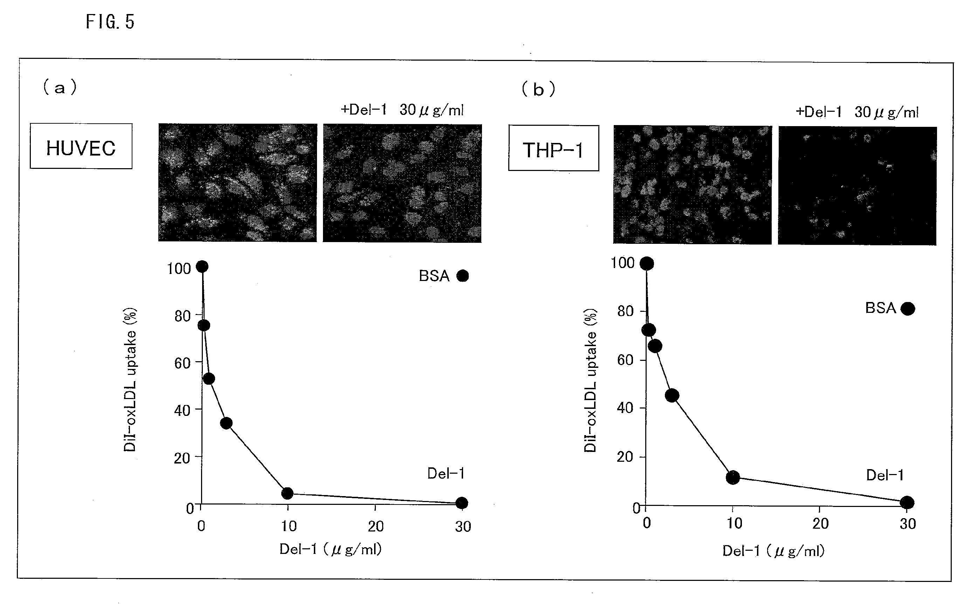

Fig. 5 is a set of drawings (a) and (b) showing results of examination of whether or not Del-1 is capable of inhibiting the uptake of oxidized LDL into human vascular endothelial cells (HUVECs) endogenously expressing oxidized LDL receptors and into macrophages (THP-1 induced to differentiate into macrophages), (a) being fluorescence micrographs and a graph as obtained with use of human vascular endothelial cells (HUVECs), (b) being fluorescence micrographs and a graph as obtained with use of macrophages (THP-1 induced to differentiate into macrophages).

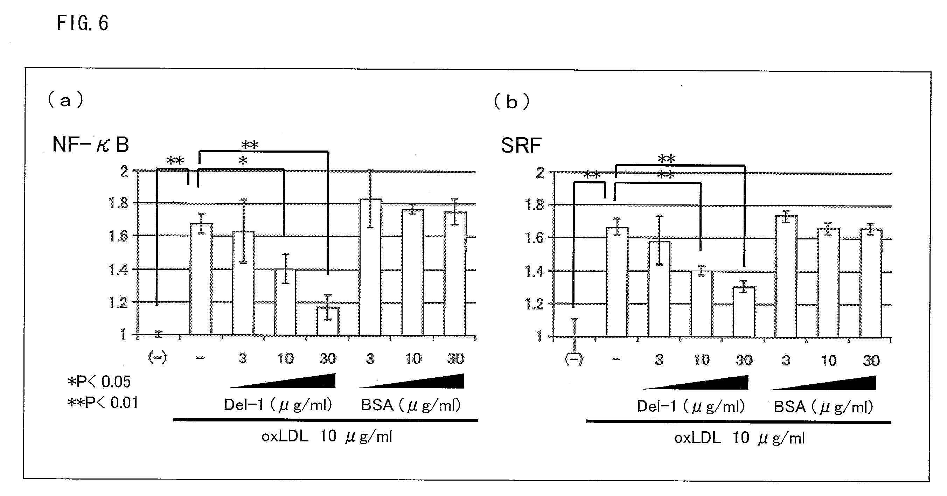

Fig. 6 is a set of drawings (a) and (b) showing results of evaluation of the inhibitory effect of Del-1 on an oxidized-LDL-dependent cellular reaction (e.g., activation of NFκB and SRF), (a) being a graph showing results of examination of activation of NFκB, (b) being a graph showing results of examination of activation of SRF.

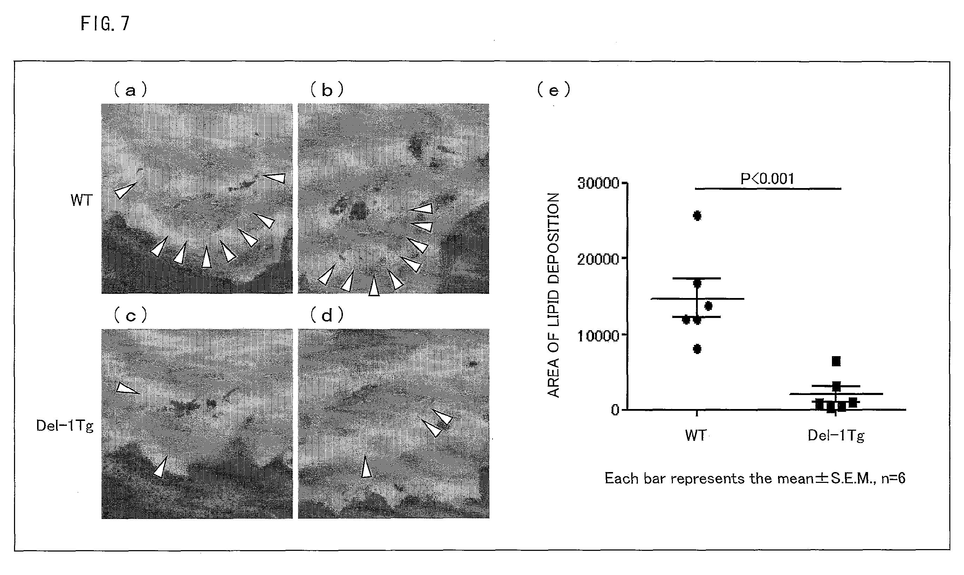

Fig. 7 is a set of drawings (a) through (d), (a) and (b) being lipid chromatic figures (Oil Red O chromatic figures) of the aortic roots of 24-week-old wild-type (indicated by "WT" in the drawings) mice freely fed with high-fat diet for 20 weeks, (c) and (d) being lipid chromatic figures (Oil Red O chromatic figures) of the aortic roots of 24-week-old Del-1-overexpressing mice (indicated by "Del-1-Tg" in the drawings), (e) being a graph showing results of quantitative determination of the areas of lipid deposition observed in the aortic roots.

### Description of Embodiments

The present invention is described in detail below. The scope of the present invention is not bound by the descriptions that follow, and embodiments and modifications other than those illustrated below may be implemented as appropriate to such an extent that the gist of the present invention is not defeated. All of the known documents described herein are incorporated herein by reference.

### [Oxidized LDL Inhibitor of the Present Invention]

An oxidized LDL inhibitor of the present invention contains a Del-1 (developmental endothelial locus-1) protein as an active ingredient.

As used herein, "Del-1" is known as a protein that is expressed and secreted in human vascular endothelial cells or in human macrophages (e.g., see Non-patent Literature 2). Fig. 1 shows a structure and amino acid sequence of Del-1. Del-1 is also called "Homo sapiens EGF-like repeats and discoidin I-like domains 3 (EDIL3)", and its amino acid sequence and nucleotide sequence are disclosed as Accession Number: NM005711 in Genbank. The amino acid sequence of Del-1 is shown in SEQ ID NO: 1, and its nucleotide sequence is shown in SEQ ID NO: 2.

Del-1 is a protein composed of 480 amino acids, and is divided into S, E1, E2, E3, C1, and C2 domains, in order of proximity to the N-terminus. "S" means a signal peptide. "E" means a EGF-like domain. "C" means a factor 5/8 C-terminal domain. Del-1 is known to bind to phosphatidyl serine (PS) especially in the C1 and C2 domains at the C-terminus to promote phagocytosis of apoptotic cells by macrophages (e.g., see Non-patent Literature 2). Known effects of Del-1 are an angiogenic effect(e.g., see Non-patent Literature 3) and an anti-inflammatory effect (e.g., see Non-patent Literature 1). Del-1 has, in its E2 domain, an RGD sequence (RGD peptide) composed of an arginine-glycine-asparatic acid. The RGD sequence is an amino acid sequence that is commonly found in proteins in which a large number of cells constitute a matrix (ECM), and is known to be closely related to cell adhesion.

The inventors of the present invention found novel effects of Del-1, i.e. the capabilities of (i) binding specifically to oxidized LDL, (ii) inhibiting the uptake of oxidized LDL into cells, and (iii) suppressing an oxidized-LDL-dependent cellular reaction, and finally accomplished an oxidized LDL inhibitor containing Del-1 as an active ingredient, based on the finding.

As used herein, the term "Del-1 protein" is meant to encompass not only (a) Del-1 consisting of an amino acid sequence of SEQ ID NO: 1, but also (b) a Del-1 variant (i) consisting of an amino acid sequence, in which one or more amino acids are substituted, deleted, inserted, or added in the amino acid sequence of SEQ ID NO: 1, and (ii) having oxidized LDL inhibitory activity. The term "Del-1 protein" also includes partial proteins of (a) and (b), provided such partial proteins have oxidized LDL inhibitory activity. Further, the term "Del-1 protein" is not limited to human-derived Del-1 of SEQ ID NO: 1, and also includes non-human mammal-derived Del-1. Examples that are applicable to the present invention are monkey-derived Del-1 (such as Genbank Accession Number: NM001132845), bovine-derived Del-1 (Genbank Accession Number: XM618255), swine-derived Del-1 (Genbank Accession Number: XM003123766), chicken-derived Del-1 (Genbank Accession Number: XM424906), rat-derived Del-1 (Genbank Accession Number: XM002728994), mouse-derived Del-1 (Genbank Accession Number: NM001037987), etc. Furthermore, the term "Del-1 protein" also includes a Del-1-like protein having an amino acid sequence other than an amino acid sequence of SEQ ID. NO: 1 and having the same activity as that of "Del-1". That is, unless otherwise specified herein, the term "Del-1" means a protein composed of amino acids of SEQ ID NO: 1, and the term "Del-1 protein" is meant to include Del-1, non-human-derived Del-1, Del-1 variants, Del-1 partial proteins, and Del-1-like proteins.

As used herein, the phrase "one or more amino acids are substituted, deleted, inserted, or added" means that such a number (preferably 10 or less, more preferably 7 or less, most preferably 5 or less) of amino acids that can be substituted, deleted, inserted, or added by a known method for the production of a variant polypeptide, such as site-directed mutagenesis, are substituted, deleted, inserted, or added. Such a variant of protein is not limited to a protein having a variant artificially introduced by a known method for the production of a variant polypeptide, but may be a protein obtained by isolating and purifying a naturally-occurring variant protein.

In the present invention, the Del-1 protein may contain an additional polypeptide. Examples of additional polypeptides include epitope tag polypeptides such as His, Myc, and Flag.

Further, in the present invention, the Del-1 protein may be one expressed in a host cell by introducing a Del-1-protein coding polynucleotide (referred to as "Del-1 gene") into the host cell, or may be one isolated from a cell, tissue, or the like and purified. Further, the Del-1 protein may be one chemically synthesized.

As used herein, the phrase "having oxidized LDL inhibitory activity" means that a Del-1 variant has activity to be capable of (i) binding specifically to oxidized LDL, (ii) inhibiting the uptake of oxidized LDL into cells, and (iii) suppressing an oxidized-LDL-dependent cellular reaction. Whether or not a Del-1 variant has oxidized LDL inhibitory activity can be confirmed by a method described in section "Examples".

Specifically, a Del-1 variant confirmed by a method described in Example 1 to be binding specifically to oxidized LDL can be determined to be capable of (i) binding specifically to oxidized LDL. Further, when it is found by a method described in Example 2 that there is a significant reduction in the uptake of oxidized LDL into cells in the presence of a Del-1 variant as compared with the uptake of oxidized LDL into cell in the absence of the Del-1 variant, the Del-1 variant can be determined to be capable of (ii) inhibiting the uptake of oxidized LDL into cells. Further, when it is found by a method described in Example 3 that there is a significant reduction in NFκB and SRF signals in the presence of a Del-1 variant as compared with NFκB and SRF signals in the absence of the Del-1 variant, the Del-1 variant can be determined to be capable of (iii) suppressing an oxidized-LDL-dependent cellular reaction. It should be noted that whether or not there is a significant reduction in data obtained in the presence of a Del-1 variant with respect to data obtained in the absence of the Del-1 variant can be determined, for example, by conducting a Student's t-test. If there is a significant reduction in the former data as compared with the latter data (at a level of significance of less than 5%), it can be determined that "there is a significant reduction".

Therefore, a person skilled in the art will understand that "(b) a Del-1 variant (i) having an amino acid sequence, in which one or more amino acids are substituted, deleted, inserted, or added in the amino acid sequence of SEQ ID NO: 1, and (ii) having oxidized LDL inhibitory activity" is described herein. Further, a person skilled in the art will be able to obtain such a Del-1 variant on the basis of the descriptions herein without undue experiment.

The amount of the Del-1 protein that is contained in the oxidized LDL inhibitor of the present invention is not particularly limited, provided that it is a sufficient amount to bring about the effects of oxidized LDL inhibitory activity, and can be determined as appropriate in consideration of the purity, dosage form, or method of intake of the Del-1 protein. For example, in the present invention, it is preferable that in order to effect sufficient oxidized LDL inhibitory activity, the Del-1 protein be contained in a percentage of 50% (w/w) or higher, more preferably 80% (w/w) or higher, even more preferably 100% (w/w), of the oxidized LDL inhibitor.

### [Pharmaceutical Composition of the Present Invention]

A pharmaceutical composition of the present invention contains the aforementioned oxidized LDL inhibitor of the present invention. The pharmaceutical composition of the present invention can both inhibit the uptake of oxidized LDL into cells and suppress an oxidized-LDL-dependent cellular reaction, and as such, the present invention is expected to be effective in treating or preventing various diseases that are caused by oxidized LDL, in particular arteriosclerotic diseases.

The pharmaceutical composition of the present invention may further contain a pharmaceutically acceptable component in addition to the oxidized LDL inhibitor (e.g., a pharmaceutically acceptable carrier etc.), provided the oxidized LDL inhibitory activity is not undermined.

The "pharmaceutically acceptable carrier" is described here. As used herein, the term "pharmaceutically acceptable carrier" (hereinafter referred to simply as "carrier") refers to a substance that is used for the purpose of aiding in drug formulation in the manufacture of a medicine or agricultural chemicals such as animal drugs and that does not have a deleterious effect on an active ingredient. Furthermore, the term is also intended to mean that there are no toxic consequences in an individual given the pharmaceutical composition of the present invention and that the carrier per se does not induce the production of a hazardous antibody.

The carrier can be selected as appropriate from among various organic or inorganic carrier substances that are usable as raw materials for drug formulation, depending on the below-mentioned forms of administration or dosage forms of the pharmaceutical composition. For example, the carrier may be combined as any of the following: an excipient, a lubricant, a binder, disintegrant etc. in a solid preparation; a solvent, a solubilizer, a suspension, a tonicity agent, a buffer, a soothing agent, etc. in a liquid preparation; an antiseptic; an antioxidant; a stabilizer; a flavoring agent; and the like. However, the present invention is not limited to these examples.

Examples of the "excipient" include lactose, saccharose, D-mannitol, xylitol, sorbitol, erythritol, starch, crystalline cellulose, etc. However, the "excipient" is not particularly limited to these examples, provided it is an excipient normally employed in the field of pharmaceuticals.

Examples of the "lubricant" include magnesium stearate, calcium stearate, wax, talc, colloid silica, etc. However, the "lubricant" is not particularly limited to these examples, provided it is a lubricant normally employed in the field of pharmaceuticals.

Examples of the "binder" include alpha starch, methyl cellulose, crystalline cellulose, saccharose, D-mannitol, trehalose, dextrin, hydroxypropylcellulose, hydroxypropyl methylcellulose, polyvinyl pyrrolidone, etc. However, the "binder" is not particularly limited to these examples, provided it is a binder normally employed in the field of pharmaceuticals.

Examples of the "disintegrant" include starch, carboxymethyl cellulose, low substituted hydroxypropylcellulose, carboxymethylcellulose calcium, croscarmellose sodium, carboxymethyl starch sodium, etc. However, the "disintegrant" is not particularly limited to these examples, provided it is a disintegrant normally employed in the field of pharmaceuticals.

Examples of the "solvent" include water for injection, alcohol, propylene glycol, macrogol, sesame oil, corn oil, tricaprilin, etc. However, the "solvent" is not particularly limited to these examples, provided it is a solvent normally employed in the field of pharmaceuticals.

Examples of the "solubilizer" include polyethylene glycol, propylene glycol, D-mannitol, trehalose, benzyl benzoate, ethanol, trisaminomethane, cholesterol, triethanolamine, sodium carbonate, sodium citrate, etc. However, the "solubilizer" is not particularly limited to these examples, provided it is a solubilizer normally employed in the field of pharmaceuticals.

Examples of the "suspension" include: surface-active agents, such as stearyl triethanolamine, sodium lauryl sulfate, lauraminopropionic acid, lecithin, benzalkonium chloride, benzethonium chloride, and glyceryl monostearate; hydrophilic polymers, such as polyvinyl alcohol, polyvinyl pyrrolidone, carboxymethylcellulose sodium, methyl cellulose, hydroxymethyl cellulose, and hydroxypropyl cellulose; and the like. However, the "suspension" is not particularly limited to these examples, provided it is a suspension normally employed in the field of pharmaceuticals.

Examples of the "tonicity agent" include sodium chloride, glycerin, D-mannitol, etc. However, the "tonicity agent" is not particularly limited to these examples, provided it is a tonicity agent normally employed in the field of pharmaceuticals.

Examples of the "buffer" include: buffers such as phosphate, acetate, carbonate, and citrate; and the like. However, the "buffer" is not particularly limited to these examples, provided it is a buffer normally employed in the field of pharmaceuticals.

Examples of the "soothing agent" include benzyl alcohol etc.. However, the "soothing agent" is not particularly limited to these examples, provided it is a soothing agent normally employed in the field of pharmaceuticals.

Examples of the "antiseptic" include p-hydroxybenzoic acid esters, chlorobutanol, benzyl alcohol, phenethyl alcohol, dehydroacetic acid, sorbic acid, etc. However, the "antiseptic" is not particularly limited to these examples, provided it is an antiseptic normally employed in the field of pharmaceuticals.

Examples of the "antioxidant" include, sulfite, ascorbic acid, etc. However, the "antioxidant" is not particularly limited to these examples, provided it is an antioxidant normally employed in the field of pharmaceuticals.

Further, the stabilizer and the favoring agent are not particularly limited, provided they are those normally employed in the field of pharmaceuticals.

A form of administration of the pharmaceutical composition according to the present invention may be oral or parenteral (e.g., intravenous, rectal, interperitoneal, intramuscular, or subcutaneous), and any suitable route of administration can be employed for any form of drug formulation. For easy administration, it is particularly preferable that the composition according to the present invention be administered orally. As used herein, the term "parenteral" refers to any mode of administration including intraventricular injections and infusions, intravenous injections and infusions, intramuscular injections and infusions, interperitoneal injections and infusions, intrasternal injections and infusions, subcutaneous injections and infusions, and intraarticular injections and infusions.

In a case where the pharmaceutical composition according to the present invention is administered orally, examples of dosage forms of such a pharmaceutical composition (hereinafter also referred to as "oral drug") include: a solid preparation, such as a powdered medicine, granules, a tablet, a liposome, or a capsule (including a soft capsule and a microcapsule), and a powdered drug; and a liquid preparation such as a syrup.

The "liquid preparation" can be prepared with the carrier by a method normally used in the field of pharmaceuticals. Examples of the carrier here include: water; an organic solvent such as glycerol, glycol, polyethylene glycol; a mixture of water and any of these organic solvents; and the like. Further, the liquid preparation may further contain a solubilizer, a buffer, a tonicity agent, a stabilizer, and/or the like.

The "solid preparation" can be prepared with the carrier by a method normally used in the field of pharmaceuticals. Examples of the carrier here include an excipient, a lubricant, a binder, a disintegrant, a stabilizer, a favoring agent, and/or the like.

In the preparation of such an oral drug, a lubricant, a glidant, a colorant, perfume, and/or the like may be further combined therewith for any purpose.

Alternatively, in a case where the pharmaceutical composition according to the present invention is administered parenterally, examples of dosage forms of such a pharmaceutical composition (hereinafter also referred to as "parenteral drug") include injections, suppositories, pellets, drops, etc. Such a parenteral drug can be prepared by, in a manner known in the field of pharmaceuticals, dissolving or suspending the pharmaceutical composition according to the present invention in a diluent (e.g., distilled water for injection, a physiological saline, an aqueous solution of glucose, vegetable oil for injection, sesame oil, soy bean oil, peanut oil, corn oil, propylene glycol, polyethylene glycol, etc.) and further adding a bactericide, a stabilizer, a tonicity agent, a soothing agent, and/or the like for any purpose.

An embodiment of the pharmaceutical composition according to the present invention may be a sustained-release preparation prepared by a technique normally employed in the field of pharmaceuticals.

The pharmaceutical composition according to the present invention may be administered alone or in combination with another drug. Examples of the administration of the pharmaceutical composition in combination with another drug include simultaneous administration of the pharmaceutical composition as a mixture with another drug, simultaneous or concurrent administration of the pharmaceutical composition as a drug separate from another drug, and administration of the pharmaceutical composition over time. However, the present invention is not limited to these examples.

Further, the number of doses of the pharmaceutical composition according to the present invention is administered per day is not particularly limited. The pharmaceutical composition according to the present invention may be administered in a single or multiple doses per day, provided the oxidized LDL inhibitor of the present invention is administered within a required daily dose range.

A person skilled in the art will easily understand that the pharmaceutical composition according to the present invention can be applied to non-human mammals (e.g., mice, rats, rabbits, dogs, cats, cows, horses, pigs, monkeys, etc.), as well as humans.

The amount of the oxidized LDL inhibitor (or Del-1 protein) in the pharmaceutical composition of the present invention is not particularly limited, provided it is a sufficient amount to bring about the effects of oxidized LDL inhibitory activity and can be determined as appropriate in consideration of the purity, dosage form, or method of intake of the Del-1 protein and the sex, age, body weight, health condition, etc. of a subject who takes in the pharmaceutical composition.

In order to bring about the effects of oxidized LDL inhibitory activity, the Del-1 protein can be contained in the pharmaceutical composition of the present invention so that an intake of the Del-1 protein is 10 mg to 3000 mg, preferably 150 mg to 1000 mg, per adult per day in dry weight.

The embodiments of the present invention are described in more detail with reference to the Examples below. Of course, the present invention is not limited to the Examples below, and there are lots of details in various aspects.

### [Examples]

### [Example 1] ELISA (Enzyme-linked Immunosorbent Assay) Evaluation of Binding of Del-1 to Oxidized LDL

### (Methodology)

### (1) Del-1

Human-derived Del-1 used in the experiments was purchased from R&D. The Del-1, obtained by expression in CHO cells followed by purification, is a recombinant protein having a His tag added to the C-terminus.

### (2) Preparation of LDL

Blood plasma was obtained by separation from blood collected from a healthy individual into a blood-collecting tube into which an ACD (acid-citrate-dextrose)-containing buffer had been added. The blood plasma had its specific gravity adjusted with the addition of potassium bromide to be 1.019, and was then centrifuged at 58,000 rpm for 20 hours. The lower layer was collected, had its specific gravity adjusted with potassium bromide to be 1.063, and was then centrifuged at 58,000 rpm for 20 hours. The ultracentrifuge used was a Beckman's L-80.

The upper layer was collected, and was dialyzed through a dialysis membrane (Slid-A-Lyzer Dialysis Cassettes 10 K MWCO; manufactured by Pierce) with an external solution (PBS (-)) (which was replaced with a fresh external solution four times) to give purified human LDL.

### (3) Preparation of Oxidized LDL

The mass of protein in the purified human LDL was measured using a BCA Protein Assay Kit (manufactured by Pierce). A solution was prepared with PBS (-) so that the concentration of the purified human LDL was 3 mg/ml. Copper sulfate was added to the solution so that the concentration of copper sulfate was 7.5 µM. The resulting solution was incubated for 16 hours in an incubator at 37°C in 5% CO₂. Then, the resulting solution was dialyzed with an external solution (which was replaced with a fresh external solution four times) to give human oxidized LDL. The external solution used was a 0.15 M sodium chloride solution containing 2 mM EDTA. In some of the drawings that show the results of the Examples, oxidized LDL is denoted by "oxLDL".

### (4) ELISA

After an anti-ApoB antibody (manufactured by Binding Site) was immobilized to each well of 384-well plates (manufactured by Greiner) and the plates were blocked with 25% Block Ace (Dainippon Sumitomo Pharma Co., Ltd.), LDL or oxidized LDL was bound to each well. After the plates were washed twice with PBS (-), Del-1 diluted with a solution (10 mM HEPES, 150 mM NaCl, pH 7.4) was added to each well, and was reacted for 1 hour at room temperature to bind to LDL or oxidized LDL. Del-1 binding to LDL or oxidized LDL was then detected with an anti-His tag antibody (Invitrogen). (a) of Fig. 2 shows a conceptual diagram explaining a principle of ELISA detection of Del-1 binding to LDL or oxidized LDL.

### (Results)

(b) of Fig. 2 shows results of the ELISA detection of Del-1 binding to LDL or oxidized LDL.

According to (b) of Fig. 2, it was confirmed that Del-1 had bound specifically to oxidized LDL at concentrations of 1 µg/ml, 3 µg/ml, and 10 µg/ml. Meanwhile, it was not found that Del-1 binds specifically to LDL.

### [Example 2] Evaluation of the Inhibitory Effect of Del-1 on the Uptake of Oxidized LDL into Cells

### (Methodology)

### (1) Preparation ofDiI-labeled LDL or DiI-labeled Oxidized LDL (DiI-oxLDL)

Human LDL or human oxidized LDL was diluted with a 0.15 M sodium chloride solution containing 2 mM EDTA to have its concentration adjusted to be 1 mg/ml. DiI (D282; manufactured by Invitrogen) and a lipoprotein-deficient serum (manufactured by Sigma) were added to the resulting solution so that their final concentrations were 0.3 mg/ml and 5 mg/ml respectively, and were reacted for 18 hours at 37°C. DiI used was in the form of a solution prepared by suspending DiI in DMSO so that its concentration was 30 µg/ml.

After the reaction, the product had its specific gravity adjusted with sodium chloride and potassium bromide to be 1.15, and was then centrifuged at 58,000 rpm for 20 hours. The upper layer was collected, and was dialyzed through a dialysis membrane (Slid-A-Lyzer Dialysis Cassettes 10 K MWCO) with an external solution (which was replaced with a fresh external solution four times) to give DiI-labeled LDL or DiI-labeled oxidized LDL. The external solution used was a 0.15 M sodium chloride solution containing 2 mM EDTA. In some of the drawings that show the results of the Examples, DiI-labeled LDL and DiI-labeled oxidized LDL are denoted by "DiI-LDL" and "DiI-oxLDL", respectively. (2) Various Type of Receptor Expression Cells

Various types of cells cultured on 10%-FBS-containing DMEM culture media (manufactured by GIBCO) for 48 hours at 37°C in 5% CO₂ were used in the Examples. Various types of receptor expression cells were prepared in the following way.

Expression vectors of various types of oxidized LDL receptors or LDL receptor expression vectors were each introduced into COS7 cells with Lipofectamin 2000 (Invitrogen). Details of the expression vectors of various types of oxidized LDL receptors or LDL receptor expression vectors are as follows:
LOX-1 expression vector (pcDNA6.2-human LOX-1-V5), SR-A expression vector (pcDNA6.2-human SR-A-V5), CD36 expression vector (pcDNA6.2-human CD36-V5), SR-BI expression vector (pcDNA6.2-human SR-BI-V5), and LDL receptor (LDLR) expression vector were prepared by cloning human LOX-1 cDNA (Genbank Accession Number: NM002543), human SR-A cDNA (Genbank Accession Number: NM002445), human CD36 cDNA (Genbank Accession Number: NM000072), human SR-BI cDNA (Genbank Accession Number: NM005505), and human LDLR cDNA (Genbank Accession Number: NM000527) from a human cDNA library and splicing them into mammalian expression vectors pcDNA6.2/V5/GW/D-TOPO (Invitrogen), respectively. It should be noted that each of the receptor had a V5 tag added to the C-terminus.

### (3) HUVECs (Human Umbilical Vein Endothelial Cells)

HUVECs were purchased from LONZA, and was cultured on an EGM-2 culture medium (manufactured by LONZA) at 37°C in 5% CO₂. HUVECs used in the experiments were those having a passage number of seven or less.

### (4) THP-1 (Human Monocytoid Leukemia Cell Line)

THP-1 was cultured on an RPMI 1940 culture medium (manufactured by GIBCO) containing 20 µM 2-mercaptoethanol and containing 10% FBS at 37°C in 5% CO₂. THP-1 cells used in the experiments were those cultured for 72 hours in the presence of 100 nM PMA (phorbol-12-myristate-13-acetate) and induced to differentiate into macrophage-like cells.

### (5) Experiment of Uptake of Oxidized LDL or LDL into Cells

After various types of cells seeded in wells of 384-well plates (manufactured by Greiner) were washed with a serum-free DMEM culture medium, a mixed solution of Del-1 and DiI-labeled LDL or DiI-labeled oxidized LDL was added to each well, and was reacted at 37°C in 5% CO₂. Fluorescence signals from DiI-labeled LDL or DiI-labeled oxidized LDL thus taken up into the cells were detected and quantitatively evaluated with an INCell Analyzer 1000 (manufactured by GE Healthcare).

### (Results)

Fig. 3 shows results of assessment of the effect of Del-1 on the uptake of oxidized LDL or LDL into LDL-receptor-expressing cells or LOX-1-expressing cells. (a) of Fig. 3 is a fluorescence micrograph of cells produced by the uptake of DiI-labeled LDL into LDL-receptor-expressing cells in the absence of Del-1, and (b) of Fig. 3 is a fluorescence micrograph of cells produced by the uptake of DiI-labeled LDL into LDL-receptor-expressing cells in the presence of Del-1 (30 µg/ml). (c) of Fig. 3 is a fluorescence micrograph of cells produced by the uptake of DiI-labeled oxidized LDL into LOX-1-expressing cells in the absence of Del-1, and (d) of Fig. 3 is a fluorescence micrograph of cells produced by the uptake of DiI-labeled oxidized LDL into LOX-1-expressing cells in the presence of Del-1 (30 µg/ml).

Further, (e) of Fig. 3 is a graph showing the fluorescence intensity of cells produced by the uptake of DiI-labeled LDL into LDL-receptor-expressing cells in the absence of Del-1 or in the presence of Del-1 (30 µg/ml) and the fluorescence intensity of cells produced by the uptake of DiI-labeled oxidized LDL into LOX-1-expressing cells in the absence of Del-1 or in the presence of Del-1 (30 µg/ml). In (e) of Fig. 3, the fluorescence intensity as obtained by the uptake of DiI-labeled LDL (or DiI-labeled oxidized LDL) in the presence of Del-1 (30 µg/ml) is indicated by a value relative to that of the fluorescence intensity (100) as obtained by the uptake ofDiI-labeled LDL (or DiI-labeled oxidized LDL) in the absence of Del-1.

An observation of the effect of Del-1 on the uptake of oxidized LDL into cells expressing LOX-1, which is an oxidized LDL receptor, showed that Del-1 inhibited the uptake of oxidized LDL into the LOX-1-expressing cells by 90% or higher at a concentration of 30 µg/ml (with a significant difference at a level of significance of less than 5% as a result of a significant difference test by Student's t-test). Meanwhile, Del-1 did not inhibit the uptake of LDL into the LDL-receptor-expressing cells (LDLR-expressing cells).

Fig. 4 is a graph showing results of examination of whether or not Del-1 is capable of inhibiting the uptake of oxidized LDL into various types of oxidized-LDL-receptor-expressing cells (LOX-1-expressing cells, SR-A-expressing cells, CD36-expressing cells, SR-BI-expressing cells).

Fig. 4 shows that Del-1 can inhibit not only the uptake of oxidized LDL into cells expressing LOX-1, but also the uptake of oxidized LDL into cells expressing another type of oxidized LDL receptor, namely SR-A, CD36, or SR-BI. It was inferred from this that Del-1 inhibits the uptake of oxidized LDL into the cells not by targeting at the oxidized LDL receptors but by targeting at oxidized LDL per se.

Fig. 5 shows results of examination of whether or not Del-1 is capable of inhibiting the uptake of oxidized LDL into human vascular endothelial cells (HUVECs) endogenously expressing oxidized LDL receptors and into macrophages (THP-1 induced to differentiate into macrophages). (a) of Fig. 5 shows results as obtained with use of human vascular endothelial cells (HUVECs), and (b) of Fig. 5 shows results as obtained with use of macrophages (THP-1 induced to differentiate into macrophages). The photographs in (a) and (b) of Fig. 5 are fluorescence micrographs. Those photographs marked with "+Del-1 30 µg/ml" are fluorescence micrographs taken with oxidized LDL taken up in the presence of Del-1 (30 µg/ml), and those photographs not marked with "+Del-1 30 µg/ml" are fluorescence micrographs taken with oxidized LDL taken up in the absence of Del-1.

Fig. 5 shows that Del-1 is capable of inhibiting not only the uptake of oxidized LDL into cells forced by gene transfer to express oxidized LDL receptors (see Fig. 4), but also the uptake of oxidized LDL into human vascular endothelial cells (HUVECs) endogenously expressing oxidized LDL receptors and into macrophages (THP-1 induced to differentiate into macrophages).

In each of the experiments shown in Fig. 5, BSA was used as a control in comparison with Del-1, but BSA was not found to inhibit uptake (see those plots marked with "BSA" in Fig. 5). Those plots marked with "BSA" in Fig. 5 each indicate the uptake amount of DiI-labeled oxidized LDL that was taken up in the presence of BSA at a concentration of 30 µg/ml.

### [Example 3] Evaluation of the Inhibitory Effect of Del-1 on an Oxidized-LDL-dependent Cellular Reaction

### (Methodology)

### (1) Reactions of cells by oxidized LDL were examined using TetOn hLOX-1-V5-His/HA-Flag hAT1/CHO cells (referred to as "LOX-1-AT1/CHO cells").

LOX-1-AT1/CHO cells were prepared in the following way. hLOX-1 expression vector (pTRE2hyg-hLOX-1) was prepared by transfecting human LOX-1 cDNA (Genbank Accession Number: NM002543) into an expression vector pTRE2hyg (manufactured by Clontech). pTRE2hyg-hLOX-1 was transferred into CHO cells with Lipofectamin 2000. For a stably expressing cell line, the cells were cultured in the presence of 100 µg/ml of G418 (manufactured by Calbiochem) and 400 µg/ml of hygromycin B (manufactured by Wako Pure Chemical Industries, Ltd.), and those ones of the cells which survived through drug selection were cloned for use in the experiments.

Into the resulting TetOn-hLOX-1-V5His cells, pTRE2hyg-HA-Flag-hAT1 and pSV2bsr (Funakoshi Corporation) were cotransfected with Lipofectamin 2000. For a stably expressing cell line, the cells were cultured in the presence of 400 µg/ml of hygromycin B (manufactured by Wako Pure Chemical Industries, Ltd.) and 10 µg/ml of blasticidin S (manufactured by Funakoshi Corporation), and those ones of the cells which survived through drug selection were cloned for use in the experiments.

The resulting LOX-1-AT1/CHO cells were cultured at 37°C in 5% CO₂ on an F12 culture medium (GIBCO) containing 10% FBS, 100 µg/ml of G418, 400 µg/ml of hygromycin B, and 10 µg/ml of blasticidin S.

### (2) Luciferase Assay

Reactions of cells by oxidized LDL were evaluated using a luciferase assay. It is well known to persons skilled in the art that NFκB and SRF are activated by oxidized LDL (e.g., see "Circulation Research. 2011; 108: 797-807, Myocardin-Related Transcription Factor A Mediates OxLDL-Induced Endothelial Injury"). Luciferase reporter vector pGF1-NFκB or pGF1-SRF (both manufactured by System Biosciences) and pRL-CMV (manufactured by Promega) were mixed, and the resulting mixture was transferred into LOX-1-AT1/CHO cells with Lipofectamin LTX (Invitrogen). Sixteen hours after the gene transfer, the culture medium was replaced with an F12 culture medium containing 0.1% of FBS and 300 ng/ml of doxycycline, and the cells were cultured for 24 hours in 0.5% CO₂.

After the cells were washed with PBS (-), 200 µl of an F12 culture medium containing 0.1% of FBS and 100 ng/ml of doxycycline were added to each well. Oxidized LDL was added in the absence or presence of Del-1, and was reacted for 20 hours in a 0.5% CO₂ incubator.

For luciferase measurement, a Dual-Luciferase Reporter Assay System (Promega) was used. Specifically, after the reacted cells were washed once with PBS (-), the cells were collected and dissolved by a passive lysis buffer and centrifuged at 10,000 rpm for 2 minutes, and the resulting supernatant was used as a sample. Luminescence intensities were measured using a luminometer Centro LB960 (manufactured by Berthold), and the value of firefly luciferase/*Cypridina* luciferase was calculated. The luminescence intensities were tabulated in graph form, assuming that the luminescence intensity measured with no addition of oxidized LDL is 1 (see Fig. 6).

### (Results)

Fig. 6 shows that in the LOX-1-AT1/CHO cells, the activation of NFκB and SRF as caused by oxidized LDL was significantly inhibited by Del-1 (at concentrations of 10 µg/ml and 30 µg/ml). Meanwhile, BSA was not found to effect such inhibition. The data shown in Fig. 6 was obtained by a significant difference test (Student's t-test). In Fig. 6, the single-asterisk mark "*" indicates a case where there was a significant difference at a level of significance of less than 5%, and the double-asterisk mark "**" indicates a case where there was a significant difference at a level of significance of less than 1%.

Del-1 was thus confirmed to be capable of inhibiting an oxidized-LDL-dependent cellular reaction.

### [Example 4] Evaluation of the Inhibitory Effect of Del-1 on Arteriosclerosis

### (Methodology)

### (1) Preparation of Del-1 Hyperexpression Mice

Del-1 hyperexpression mice (referred to as "Del-1-Tg mice") were prepared in the following way.

Human Del-1 expression vector pcDNA6.2-hDel-1 was prepared by cloning human Del-1 gene (SEQ ID NO: 2) from human brain cDNA and splicing it into a mammalian expression vector pcDNA6.2/V5/GW/D-TOPO (Invitrogen).

Del-1-Tg mice was prepared by an improved method of Gordon et al. (1980. Proc.Nalt.Acad.Sci. 77:7380-7384). That is, a DNA fragment containing a CMV promoter and a Del-1 gene was prepared by treating human Del-1 expression vector (pcDNA6.2-hDel-1) with restriction enzymes (MfeI, SmaI). The DNA fragment was microinjected into the male pronucleus of a pronuclear embryo of C57BL/6JJcl. The DNA-injected embryo was transplanted into the fallopian tube of a pseudopregnant female mouse (ICR mouse) under anesthesia, and the female mouse was allowed to naturally deliver baby mice (Del-1-Tg mice). The baby mice served as primary mice (Founder).

### (2) Lipid Staining

Twenty-four-week-old Del-1 hyperexpression mice (referred to as "Del-1-Tg mice") and wild-type mice were freely fed with high-fat diet (marketed as Atherogenic Rodoent Diet (Model Number: D12336), Research Diets) for 20 weeks. These mice were dissected to have their aortas removed from them, and lipids in the aortas were stained with an Oil Red O stain solution (manufactured by Wako Pure Chemical Industries, Ltd.). Specifics are as follows:
The mice subjected to 20 weeks of high-fat diet had their abdomens and chests opened under inhalation anesthesia with isoflurane. Aortas of the mice were perfused with a physiological saline (manufactured by Otsuka Pharmaceutical Co., Ltd.), and then removed from the mice. The aortas thus removed had their surrounding tissues manually ablated in a physiological saline, and were then fixed with 4%(v/v) paraformaldehyde-PBS. After the aortas thus fixed were washed with 60%(v/v) isopropanol to equilibrate, lipids deposited in the aortas were stained with an Oil Red O stain solution (manufactured by Wako Pure Chemical Industries, Ltd.), and were then destained with 60%(v/v) isopropanol.

The tissues thus stained were observed by using optical microscope.

### (Results)

The results are shown in Fig. 7. (a) and (b) of Fig. 7 are lipid chromatic figures (Oil Red O chromatic figures) of the aortic roots of 24-week-old wild-type (indicated by "WT" in the drawings) mice freely fed with high-fat diet for 20 weeks. (c) and (d) of Fig. 7 are lipid chromatic figures (Oil Red O chromatic figures) of the aortic roots of 24-week-old Del-1-overexpressing mice (indicated by "Del-1-Tg" in the drawings). In (a) through (d) of Fig. 7, the positive staining sites are indicated by arrows. Further, (e) of Fig. 7 is a graph showing results of quantitative determination of the areas of lipid deposition observed in the aortic roots.

It is found from Fig. 7 that the Del-1-Tg mice showed a clear reduction in lipid deposition as compared with the wild-type mice (P<0.001). It was thus confirmed that the development of arteriosclerosis can be suppressed by overexpressing Del-1 in mice. That is, it can be said that there is a possibility that cardiovascular diseases such as arteriosclerosis can be suppressed by pharmacologically using the Del-1 protein.

### Industrial Applicability

As described above, the present invention makes it possible to provide an oxidized LDL inhibitor that is capable of (i) binding specifically to oxidized LDL, (ii) inhibiting the uptake of oxidized LDL into cells, and (iii) suppressing an oxidized-LDL-dependent cellular reaction. As such, the oxidized LDL inhibitor of the present invention can serve as effective means for treating or preventing arteriosclerotic diseases that are caused by oxidized LDL.

Therefore, the present invention is applicable in medical and pharmaceutical industries.

## Claims

1. An oxidized LDL inhibitor comprising a Del-1 (developmental endothelial locus-1) protein as an active ingredient.

2. The oxidized LDL inhibitor as set forth in claim 1, wherein the Del-1 protein is (a) a protein consisting of the amino acid sequence of SEQ ID NO: 1 or (b) a protein (i) consisting of an amino acid sequence, in which one or more amino acids are substituted, deleted, inserted, or added in the amino acid sequence of SEQ ID NO: 1, and (ii) having oxidized LDL inhibitory activity.

3. A pharmaceutical composition comprising an oxidized LDL inhibitor as set forth in claim 1 or 2.
